Europäisches Patentamt

European Patent Office

Office européen des brevets

(11) Publication number: **0 468 596 B1**

## EUROPEAN PATENT SPECIFICATION

(45) Date of publication of patent specification: **12.07.95**

(51) Int. Cl.⁶: **A23K 3/03**, C12N 9/24, A23K 1/165

(21) Application number: **91201943.7**

(22) Date of filing: **24.07.91**

(83) Declaration under Rule 28(4) EPC (expert solution)

(54) **Enzymatic treatment of silage.**

(30) Priority: **24.07.90 EP 90202020**

(43) Date of publication of application:
**29.01.92 Bulletin 92/05**

(45) Publication of the grant of the patent:
**12.07.95 Bulletin 95/28**

(84) Designated Contracting States:
**AT BE CH DE DK ES FR GB GR IT LI LU NL SE**

(56) References cited:
**WO-A-89/10066**

**Chemical Abstracts, vol. 87, no. 22, November 28, 1977, Colombus, Ohio US; abstract no. 182914, SILVERS V.S. et al. "Use of hydrolytic enzyme preparations during ensilage of corn cobs"**

**Chemical Abstracts, vol. 94, no. 11, March 16, 1981, Columbus, Ohio, SU; abstract no. 82520X, BODGAN S.D. et al.: Use of enzymic preparations for the ensilage of wheat straw"**

(73) Proprietor: **GIST-BROCADES N.V.**
**Wateringseweg 1**
**P.O. Box 1**
**NL-2600 MA Delft (NL)**

(72) Inventor: **Harder, Abraham**
**De Vaert 9**
**NL-2651 EP Berkel en Rodenrijs (NL)**

(74) Representative: **Matulewicz, Emil Rudolf Antonius, Dr. et al**
**Gist-Brocades NV**
**Patents and Trademarks Department**
**Wateringseweg 1**
**P.O. Box 1**
**NL-2600 MA Delft (NL)**

Chemical Abstracts, vol. 93, no. 3, July 21, 1980, Columbus, Ohio, US; abstract no. 24758Z, TASHPULATOV, ZH: "Microbial processes in the ensiling of guza-paya using cellulolytic enzymes"

O, Jorgensen et al., In.: "Enzyme systems for lignocellulose degradation, Ed. M.P. Coughlan" 1989, ELSEVIER APPLIED SCIENCE, LONFON, GB

Chemical Abstracts, vol. 110, no. 25, June 25, 1989, Columbus, Ohio, US; abstract no. 230420F, NAKASHIMA Y. et al.: "Rumen degradation of straw.6.Effect of polysaccharidase enzymes on degradation characteristics of ensiled rice straw"

**Description**

The present invention relates to the field of agriculture, more specifically, the present invention relates to an improvement in the enzymatic treatment of fodder silage.

Background of the Invention

Improvements in silage technology have, next to natural and artificial drying, made ensiling the most widely applied technology for the preservation of forage. It has been estimated that in western Europe, 60% (approximately 77 million tons dry matter) of the forage preserved for winter is ensiled. In the U.S., approximately 80 million tons (dry matter) are ensiled per year. Ensiling is also widely used in eastern Europe and Canada.

However, effluent production and nutrient losses in silage caused by fermentation (especially butyric acid fermentation) and aerobic deterioration have become increasingly problematic with the growing demand for higher efficiency in animal livestock production. These problems often depend on factors such as the type of crop, climactic conditions and available technology. Recently introduced production limits for dairy farming and, in some countries, environmental legislation indirectly restricting the type and amounts of nutrients used for livestock production emphasize the need to feed high quality silages (Spoelstra, S.F. (1991) In: Proceedings of the EGF Conference - "Forage Conservation Towards 2000"; Braunschweig, Germany).

Grasses (such as rye-grasses, etc.), cereals (such as maize, sorghum, wheat, etc.) and legumes (such as alfalfa, clover, etc.) are the primary crops used as fodder for ensiling. Beet tops and sugar beet pulp are also used in relatively smaller amounts.

Maize ensiles well, but is susceptible to aerobic deterioration. Grass, mainly perennial rye-grass, is often heavily fertilized and has, as a result, a low ratio of water soluble carbohydrates to buffering capacity making it susceptible, after ensiling, to butyric acid fermentation. Butyric acid fermentation leads to dry matter losses in the silage, thus lowering the nutritional value and consequently, livestock production efficiency.

To avoid undesirable butyric acid fermentation, the grass is normally wilted in the field to, on average, 500 g DM (dry matter) per kg. Alternatively, at DM contents below 350 g DM per kg, additives such as enzymes are applied.

Continuing research efforts have been undertaken to study whether silage preservation is enhanced and silage intake and digestibility is improved by the addition of cell wall degrading enzymes, cellulases and hemicellulases, in particular.

The actions of cellulases and hemicellulases liberate water soluble carbohydrates from the structural polysaccharides of the plant cell wall, thus providing substrates for the production of lactic acid via fermentation by the lactic acid bacteria present in the silage. These enzymes are probably also responsible for the biolytic disruption of the plant cell walls, releasing further water soluble carbohydrates and other materials from within the cell for use by the lactic acid bacteria. In addition, the pre-digestion of the plant cell walls during the storage of the silage may lead to their being more rapidly degraded in the rumen and thus enhance digestibility and moreover may provide increased amounts of liberated carbohydrates for added nutritional value for the animal ingesting the thus-treated silage.

The production of lactic acid (as well as acetic acid) by lactic acid bacteria present in the silage is also responsible for the lowering of the pH of the silage. The lowering of the pH, generally decreasing to about pH 4.5 and may be as low as about pH 4, creates an unfavorable environment for the growth of yeasts, as well as many other undesired microbes such as butyric acid-producing microorganisms, thus preserving the silage and maintaining its nutritional content.

Additionally, the lactic acid bacteria also serve as probiotics, beneficially enhancing the intestinal flora of the animal ingesting the silage.

Most, if not all enzyme preparations investigated for use in silage are rather crude fermentation products from fungi. These enzyme preparations contain many different enzyme activities.

For example, a cellulase preparation, obtained from a Penicillium verruculosum mutant strain, is disclosed in East German patent publication DD-278359 (published May 2, 1990) to be applicable for partial or total hydrolysis of cellulose and hemicellulose in industrial processes such as the production of silage, inter alia. The cellulase preparation is purported to contain high cellulolytic activity combined with beta-glucosidase, xylanase and amylase activity.

A probiotic inoculum for silage comprising lactic acid bacteria transformed with exogenous DNA encoding an enzyme capable of degrading polysaccharides and oligosaccharides in a silage crop to provide a source of water soluble carbohydrates for the bacteria themselves is described in PCT Application WO 89/01970 (published March 9, 1989).

"Chemical Abstracts (CA) vol. 87 no. 22, 28.11.77, Columbus, Ohio, U.S., Abstract no. 182914c describes the treatment of corn cobs with hydrolytic enzymes.

CA vol. 94 no. 11, 16.03.81, abstract no. 82520x describes the treatment of wheat straw with enzymes.

CA vol. 93 no. 3, 21.06.80 abstract no. 24758z describes the use of cellulolytic enzymes in the ensiling of guza-paya in the presence of cellolignorin.Px.

CA vol. 110, no.25, 25.06.89 abstract no. 230420f describes the use of glucanase, xylanase and both endo- and exocellulase on rice straw.

O. Jorgensen et al. "Enzyme systems for lignocellulose degradation" 1989, Elsevier, Applied Science, London 6b pages 347-355 discloses that hemicelluloses should be used as part of a cellulase complex consisting of endo- and exo-activities including $\beta$-glucosidase, in order to obtain optimal silage.

## Summary of the Invention

According to the present invention, it has been found that endo-xylanase activity, of the many enzymatic activities present in enzymatic preparations used to ensile fodder, is particularly responsible for the release of water soluble carbohydrates, which through the fermentative action of lactic acid bacteria, leads to the preservation and improved nutritional quality of the silage. Furthermore, it has been found that supplementing fodder to be ensiled with sufficient amounts of endo-xylanase, without the presence of other enzymatic activities, provides optimal preservation and nutritional quality of the thus-treated silage. It has also been observed that animals ingest greater amounts of silage preserved in this manner, as opposed to silage which has deteriorated. These factors all lead to an enhancement in the efficiency of livestock production.

The present invention thus provides a silage composition supplemented with an amount of endo-xylanase activity effective in releasing water soluble carbohydrates from the ensiled plant cell material (fodder), thus enhancing the production of lactic acid and acetic acid by lactic acid bacteria, which in turn improves the preservation and nutritional quality of the silage. The endo-xylanase is added in a form which is essentially free of other enzymatic activity.

As the fodder to be ensiled does not always contain sufficient amounts of lactic acid bacteria to achieve proper ensiling, the present invention also provides silage compositions which further contain an inoculum of a lactic acid bacteria, included as a supplement in an amount sufficient to assist the preservation of the silage and to act as probiotics for the enhancement of the intestinal flora of the animal ingesting the thus-treated silage composition.

It is also an object of the present invention to provide a method of preserving silage characterized in that the silage is supplemented with an amount of endo-xylanase activity effective in increasing the amount of water soluble carbohydrates present in the silage for use in the production of lactic acid and acetic acid by lactic acid bacteria.

It is a further object of the present invention to provide a method for the improvement of the nutritional value of silage by supplementing the silage with an amount of endo-xylanase activity effective in releasing water soluble carbohydrates from the ensiled plant cell material for utilization by the animal ingesting the silage. The endo-xylanase present in the silage, also provides a supplement of an essential enzyme for the animal itself.

It is yet another object of the present invention to provide a method for improving the in vivo (dry matter) digestibility of silage in animals wherein the animal is fed a diet consisting at least in part of a silage which has been supplemented with an amount of endo-xylanase activity effective in at least partially degrading, and thus pre-digesting, plant cell walls.

## Brief Description of the Figure

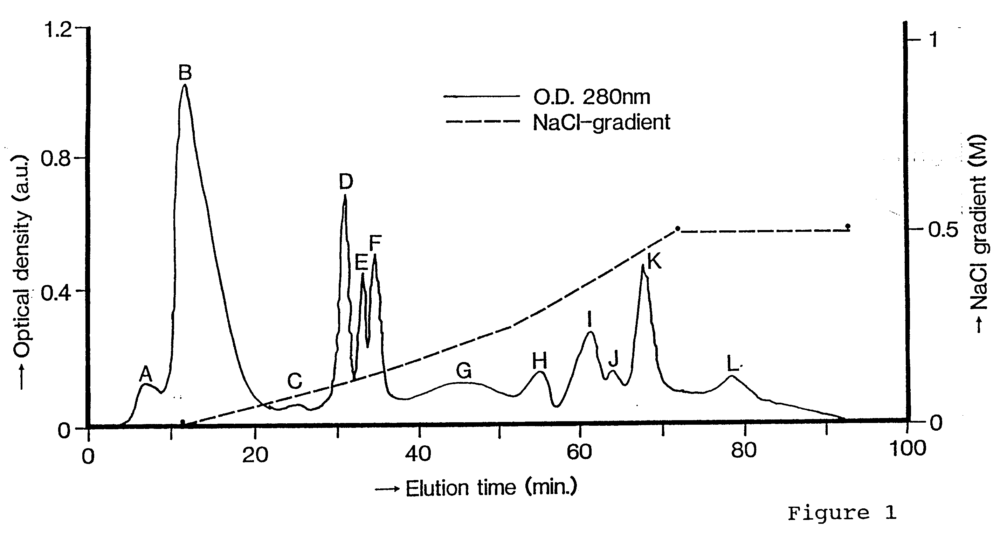

Figure 1: HPLC elution profile of a culture filtrate obtained from Aspergillus niger DS16813 (CBS 323.90). This strain was later reclassified as more likely belonging to the species Aspergillus tubigensis.

## Detailed Description of the Invention

According to the present invention, fodder to be ensiled is supplemented with an amount of endo-xylanase which is effective in producing a silage having a significant increase in the amount of water soluble carbohydrates for use by the lactic acid bacteria or as nutrients for the animal ingesting the silage; and improved qualities such as increased fermentative production of lactic acid and acetic acid (lactic acid being produced in greater quantities relative to acetic acid) by (probiotic) lactic acid bacteria and the consequent lowering of pH which serves to preserve the silage composition and its nutritional value, these factors contributing to the improvement of livestock production efficiency. Until the present invention, endo-xylanases were not known to be specifically responsible for such a significant role in the production of improved silages. Nor was it known that the addition of an endo-xylanase in a form which is substantially free of other enzymatic activity (especially other cell wall degrading enzymatic activity) to fodder to be ensiled, would lead to obtaining a silage which is not only better preserved, but has improved nutritional quality as well.

Silage compositions, according to the present invention contain more than 50,000 units endo-xylanase activity per kg (fresh weight) fodder. The endo-xylanase optimally has a pH optimum in the range of pH 3.5 to pH 6.0 in order to ensure that maximum enzyme efficacy is obtained at the acidic pH conditions which exist in the process of ensiling fodder. As stated above, the endo-xylanase is added in a form which is substantially free of other enzymatic activity in general, and cell wall degrading enzymatic activity in particular.

The supplementation of the fodder to be ensiled with endo-xylanase activity which is substantially free of other enzymatic activities is also commercially attractive as a more cost-effective alternative to the use of enzyme mixtures wherein secondary enzyme activities often must be supplemented to obtain the desired effect. The phrase "substantially free of other enzymes", as defined in context of the present invention, intends that other enzymatic activity is not present in sufficient quantities to have any effect on the fodder which is used in the preparation of the silage.

For example, the endo-xylanase activity may be produced in large quantities using recombinant DNA techniques, such as described in European Patent Application no. 90202020.5 (filed July 24, 1990), the disclosure of which is hereby incorporated by reference. Alternatively, the production of endo-xylanases may be enhanced by adjusting the fermentation conditions of the endo-xylanase-producing microorganism such as the use of an inducing media containing xylan.

Endo-xylanases, for use in the context of the present invention, are preferably obtained from a filamentous fungus, more preferably from a filamentous fungus selected from the genera consisting of Aspergillus, Disporotrichum, Penicillium, Neurospora, Fusarium and Trichoderma, and most preferably obtained from a filamentous fungus selected from the species consisting of Aspergillus niger, Aspergillus awamori, Aspergillus aculeatus, Aspergillus tubigensis, Disporotrichum dimorphosporum and Trichoderma reesei.

An endo-xylanase to be used in the present invention may be identified via assay methods not critical to the present invention, such as a spot test assay. According to this method, a filtrate obtained from the culturing of a microorganism induced (e.g. with oat spelt xylan) to produce an endo-xylanase may be tested for the presence of endo-xylanase activity. Drops of the elution fractions are placed individually onto an agar film containing a citrate-phosphate buffer (see Example 1.1, below) and oat spelt xylan. The film is then incubated. If endo-xylanase activity is present, the locations of the individual drops on the agar film are visibly clear.

Endo-xylanase activity may also be identified by subjecting a xylan-containing solution to an enzyme solution suspected of containing endo-xylanase activity and spectrophotometrically analyzing the reducing sugars by the method as described by Leathers, T.D. et al. (1984) Biotechnol. Bioeng. Symp., **14**, 225.

A unit of endo-xylanase activity is herein defined as the amount ($\mu$mol) of xylose equivalents liberated per minute per mg protein at a temperature of 39°C and at pH 3.5. Protein determination was performed according to the method as described by Bradford, M.M. (1976) Anal. Biochem., **72**, 248. Bovine immuno-gamma-globulin (BIgG) was used as the protein standard.

Once identified, an endo-xylanase-producing organism may be fermented under conditions conducive to the production of endo-xylanase and the desired enzymatic activity may be isolated from the culture filtrate of the production organism and, if desired, purified by conventional means not critical to the present invention. For example, affinity and/or ion exchange chromatography techniques may advantageously be used for the preparative purification of an endo-xylanase from a culture filtrate.

Another embodiment of the present invention also envisions the supplementation of commercial enzyme preparations with endo-xylanase activity. Preferably, an amount of endo-xylanase is added to provide a total endo-xylanase activity in the final mixture of more than 50,000 units/kg fresh weight fodder.

In yet another embodiment of the present invention silage compositions are provided which further contain an inoculum of a lactic acid bacteria, included as a supplement to assist in the preservation of the silage. The lactic acid bacteria is preferably selected from the group consisting of the genera Lactobacillus, Enterococcus, Lactococcus, Pediococcus and Leuconostoc. According to the present invention, the most preferred lactic acid bacteria is selected from the group consisting of Lactobacillus plantarum, Lactobacillus acidophilus, Lactobacillus brevis, Lactobacillus bulgaricus, Lactobacillus fermentum and Lactobacillus lactis.

The supplementation of endo-xylanase to silage, according to the present invention may be applied to all ensilable plant material such as grasses, cereals and legumes, as known to those skilled in the art. The fodder normally, however, will have a dry matter (DM) content of below 350 g DM per kg fodder (fresh weight) to allow the enzyme free access to the plant cell material.

The following examples are provided so as to give those of ordinary skill in the art a complete disclosure and description of how to make and use the invention. Efforts have been made to ensure accuracy with respect to numbers used (e.g., amounts, temperature, pH, etc.) but some experimental errors and deviation should be accounted for. Unless indicated otherwise, temperature is in degrees Celsius and pressure is at or near atmospheric.

Example 1

Purification of Aspergillus tubigensis endo-xylanase.

A culture filtrate was obtained by the culturing of Aspergillus niger DS16813 (CBS 323.90, deposited at the Centraal Bureau voor Schimmelcultures, Baarn, The Netherlands on July 20, 1990 - later reclassified as more likely belonging to the species A. tubigensis; Kusters-van Someren et al. (1991) Curr. Genet. **19**, 21) in a medium containing (per liter): 30 g oat spelt xylan (Sigma); 7.5 g $NH_4NO_3$, 0.5 g KCl, 0.5 g $MgSO_4$, 15 g $KH_2PO_4$, and 0.5 g yeast extract (pH 6.0). The culture filtrate was concentrated to a volume of approximately 35 ml which was then was ultrafiltered on a Diaflo PM 10 filter in a 50 ml Amicon module to remove salts.

The supernatant was then concentrated to a volume of 10 ml and the retentate was washed twice with 25 ml 25 mM Tris-HCl buffer (pH 7.0). After washing, the retentate volume was brought to 25 ml.

This retentate was injected in 1 ml quantities onto a Syn Chropak AX 300 column (dimensions 10 x 250 mm) and eluted in the following HPLC regime:

elution rate: 2 ml/min.

elution buffer A: 25 mM Tris-HCl pH 7.0

elution buffer B: 25 mM Tris-HCl pH 7.0 + 1 M NaCl

elution gradient:

| time (min) | %A | %B |
|---|---|---|
| 0 | 99 | 1 |
| 12 | 97 | 3 |
| 30 | 80 | 20 |
| 50 | 50 | 50 |
| 70 | 0 | 100 |
| 90 | 0 | 100 |
| 95 | 99 | 1 |

Fractions of 1 ml each were collected. Detection of the eluted protein was performed by continuous measurement of the UV absorption at 280 nm. The elution profile is shown in Figure 1.

The fractions were tested for the presence of endo-xylanase activity by a spot test. This spot test consists of adding 12 ml citrate-phosphate buffer (prepared by mixing 900 ml 0.2 M $Na_2HPO_4$ and 125 ml 0.5 M citric acid, followed by an adjustment of the pH of the solution to pH 5.6 using 0.5 M citric acid or 0.2 M $Na_2HPO_4$) containing 0.5% oat spelt xylan (Sigma) to 180 mg agar (Difco) and heating the mixture to 100°C to dissolve the agar. After cooling to 60°C, the agar mixture is poured evenly onto Agarose gel-bond film. Drops of the elution fractions are placed individually onto the film and incubated for 30 min. at 30°C. If endo-xylanase activity is present, the location of the individual drops on the agar film is clear.

Total xylanase activity in the collected fractions was quantitatively determined by measuring the amount of reducing sugars produced over a predetermined time period in the microassay as described by Leathers et al. (1984), using oat spelt xylan in 50 mM sodium acetate at pH 5.0 as a substrate. Activity units are also as defined by Leathers (supra).

Exo-xylanase activity in the eluted fractions was determined by the method described by Poutanen and Puls (1988), using p-nitro-phenyl-$\beta$-D-xylopyranoside (0.3 mM, Sigma) as a substrate at pH 5.0 and 30°C.

The spot test revealed that the elution fractions corresponding to peaks B, F and K (see Figure 1) contain endo-xylanase activity. The total xylanase assay showed activity in the elution fractions of peaks B, F, H and K. The elution fractions of peaks B and H were determined to contain exo-xylanase activity.

The elution fractions of peaks F (XYL2 protein) and K (XYL A protein) were further purified by repeated ion exchange chromatography.

Example 2

Perennial English rye-grass was mown, dried for 24 hours until reaching a dry matter (DM) content of about 25%, cut into lengths of 1-2 cm and ensiled in 1 liter laboratory silos and stored for 3 months at ambient temperature before analysis.

The enzyme preparation Cellulase ABG-7 was added at a concentration of 0.2 g of protein per kg fresh weight grass (equivalent to 3200 units of endo-xylanase activity per kg fodder; protein content determined using the method as described by Bradford, M.M. (supra)). Cellulase ABG-7 is a commercial enzyme preparation produced by Trichoderma reesei and is available under the name MAXAZYM® CL-2000 (IBIS, N.V., Rijswijk, The Netherlands). The enzymatic activities of this product are summarized in Table 1, below.

Table 1

| Enzymatic activities of Cellulase ABG-7 | | |
|---|---|---|
| ACTIVITY | MEASURED PRODUCT | SPECIFIC ACTIVITY $\mu$moles/min.Mg protein |
| CMC-ase | glucose equivalent | 0.79 |
| Avicelase | glucose equivalents | 0.11 |
| Endoxylanase | xylose equivalents | 16.0 |
| Exoxylanase | p-nitrophenol | 0.48 |
| Exoarabinase | p-nitrophenol | 0.51 |
| Acetylesterase | p-nitrophenol | 0.16 |
| Polygalacturonase | galactose equivalents | 3.56 |
| Glucuronidase | p-nitrophenol | 0.003 |
| $\alpha$-Amylase | glucose | 0.25 |

The results of the silage experiment are presented in Table 2, below.

Table 2

| Mean (N = 3) composition of grass, control silage and enzyme-treated silage after incubation with Cellulase ABG-7 | | | |
|---|---|---|---|
| | GRASS | SILAGE | |
| | | CONTROL | CELLULASE ABG-7 |
| Dry matter (DM) (g/kg) | 273 | 269 | 259 * |
| Ash (g/kg DM) | 124 | 124 | 136 * |
| Lactic acid (g/kg DM) | - | 45.5 | 59.4* |
| Ethanol (g/kg DM) | - | 7.33 | 19.1* |
| Acetic acid (g/kg DM) | - | 46.9 | 82.8* |
| Crude fiber (g/kg DM) | 258 | 262 | 194 * |
| Neutral Detergent Fiber (g/kg DM) | 523 | 446 | 351 * |
| Acid Detergent Fiber (g/kg DM) | 279 | 281 | 204 * |
| Acid Detergent Lignin (g/kg DM) | 17.5 | 15.9 | 18.5* |
| Weight loss (g/kg DM) | - | 49.9 | 76.3* |
| $NH_3$-N/total N (wt% N-total) | - | 26 | 20 * |
| pH | - | 5.42 | 4.72 |

* statistically different from the control.
P < 0.05 (Student T-test)

Cellulase ABG-7 hydrolysed a large proportion of cell wall constituents to fermentable carbohydrates. This resulted in increased silage quality as characterized by the liberation of water soluble carbohydrates. This is evidenced by the increased lactic acid concentration and lower crude fiber content, lower Neutral Detergent Fibre content, lower Acid Detergent Fiber content, as well as lower pH and ammonia values in comparison to the control.

Example 3

Perennial English rye-grass was prepared as described in Example 2, above, and ensiled in 1 liter laboratory silos and stored for 3 months at ambient temperature before analysis.

The enzyme Cellulase ABG-7 (see Table 1, above) was added at concentrations of 0.005 g of protein (80 units endo-xylanase activity) per kg fresh weight grass; 0.025 g of protein (400 units endo-xylanase activity) per kg fresh weight grass; 0.050 g of protein (800 units endo-xylanase activity) per kg fresh weight grass; 0.075 g of protein (1200 units endo-xylanase activity) per kg of fresh weight grass (all protein content determined using the method as described by Bradford, M.M. (supra)).

EP 0 468 596 B1

Table 3    :    Mean (N=3) composition of grass and silages.

| | | GRASS | CONTROL SILAGE | SILAGE | | | |
|---|---|---|---|---|---|---|---|
| | | | | CELLULASE ABG-7 (g protein/kg grass) | | | |
| | | | | 0.005 | 0.025 | 0.050 | 0.075 |
| Dry matter (DM) | (g/kg) | 233 | 223 | 223 | 221 * | 219 * | 219 * |
| Ash | (g/kg DM) | 111 | | | | | |
| Lactic acid | (g/kg DM) | — | 117 | 102 | 116 | 126 | 120 |
| Ethanol | (g/kg DM) | — | 5.73 | 4.22* | 4.44* | 4.97* | 4.95* |
| Acetic acid | (g/kg DM) | — | 33.7 | 26.5 * | 20.0 * | 32.6 | 34.5 |
| Crude fibre | (g/kg DM) | 256 | 249 | 252 | 247 | 242 | 233 * |
| NDF | (g/kg DM) | 505 | 428 | 430 | 427 | 413 * | 403 * |
| ADF | (g/kg DM) | 275 | 268 | 266 | 261 | 257 * | 254 * |
| ADL | (g/kg DM) | 19.7 | 20.8 | 20.4 | 21.8 | 20.8 | 20.7 |
| Weight loss | (g/kg DM) | — | 39.9 | 26.9 * | 25.8 * | 34.7 | 36.8 |
| $NH_3$-N/total N | (wt % N-total) | — | 16 | 14 * | 11 * | 14 * | 15 |
| pH | | — | 4.25 | 4.09* | 3.93* | 4.10* | 4.11* |

* statistically different from the control.  P < 0.05 (Student T-test)

Example 4

Perennial English rye-grass was prepared as described in Example 2, above, and ensiled in 1 liter laboratory silos and stored for 3 months at ambient temperature before analysis.

In this experiment, a purified endoxylanase from Aspergillus tubigensis (specific activity 5000 units endoxylanase activity per kg fodder (Bradford protein determination method, supra); see Example 1) was combined with the Cellulase ABG-7 preparation to increase the endoxylanase activity in the preparation and added to the grass. The endoxylanase concentration in this combined enzyme product added to the grass was 0.050 g of protein (250,000 units endo-xylanase activity) per kg (fresh weight) grass and the Cellulase ABG-7 concentration added was 0.200 g of protein (3200 units endo-xylanase activity) per kg (fresh weight) grass (protein contents determined using the method as described by Bradford, M.M. (supra)).

The results are presented in Table 4.

Table 4

| Mean (N = 3) composition of grass and silages. | | | | |
|---|---|---|---|---|
| | GRASS | SILAGE | | |
| | | CONTROL | CELLULASE ABG-7 | CELLULASE ENDOXY-LANASE |
| Dry matter (DM) (g/kg) | 273 | 269 | 259 * | 259 * |
| Ash (g/kg DM) | 124 | 124 | 136 * | 133 * |
| Lactic acid (g/kg DM) | - | 45.5 | 59.4 * | 101 * |
| Ethanol (g/kg DM) | - | 7.33 | 19.1 * | 7.81* |
| Acetic acid (g/kg DM) | - | 46.9 | 82.8 * | 46.6 * |
| Crude fibre (g/kg DM) | 258 | 262 | 194 * | 192 * |
| NDF (g/kg DM) | 523 | 446 | 351 * | 344 * |
| ADF (g/kg DM) | 279 | 281 | 204 * | 205 * |
| ADL (g/kg DM) | 17.5 | 15.9 | 18.5 * | 17.3 |
| Weight loss (g/kg DM) | - | 49.9 | 76.3 * | 46.4 |
| $NH_3$-N/total N (wt % N-total) | - | 26 | 20 * | 20 * |
| pH | - | 5.42 | 4.72* | 4.39* |

* statistically different from the control. P < 0.05

Surprisingly, the addition of endo-xylanase to the Cellulase ABG-7 preparation resulted in a much higher concentration of lactic acid in the ensiled grass and subsequently a much lower pH.

Moreover the fermentation of ethanol and acetic acid is suppressed as well as the weight loss due to the formation of $CO_2$ and volatile fatty acids.

Example 5

Perennial English rye-grass was prepared as described in Example 2, above, and ensiled in 1 liter laboratory silos and stored for 3 months at ambient temperature before analysis.

Purified endoxylanase was added to the grass in concentrations ranging of 0.010 g, 0.050 g and 0.200 g of protein per kg fresh weight fodder (Bradford protein determination method, supra).

The results are presented in Table 5.

Table 5 : Mean (N=3) composition of grass and silages.

| | GRASS | CONTROL SILAGE | SILAGE ENDOXYLANASE (units/kg grass) | | |
|---|---|---|---|---|---|
| | | | 50,000 | 250,000 | 1 x 10⁶ |
| Dry matter (DM) (g/kg) | 273 | 269 | 263 | 267 | 264 |
| Ash (g/kg DM) | 124 | 124 | 126 | 127* | 130* |
| Lactic acid (g/kg DM) | - | 45.5 | 50.0 | 67.4* | 90.2* |
| Ethanol (g/kg DM) | - | 7.33 | 6.54 | 7.02 | 7.63 |
| Acetic acid (g/kg DM) | - | 46.9 | 48.2 | 53.2* | 55.9* |
| Crude fibre (g/kg DM) | 258 | 262 | 263 | 246 | 219* |
| NDF (g/kg DM) | 523 | 446 | 438 | 417* | 374* |
| ADF (g/kg DM) | 279 | 281 | 283 | 267* | 236* |
| ADL (g/kg DM) | 17.5 | 15.9 | 16.8 | 16.8 | 16.7 |
| Weight loss (g/kg DM) | - | 49.9 | 51.4 | 54.0* | 53.2* |
| NH₃-N/total N (wt % N-total) | - | 26 | 27 | 27 | 27 |
| pH | - | 5.42 | 5.23 | 5.03* | 4.70* |

* statistically different from the control. P < 0.05 (Student T-test)

The results presented in Table 5 clearly demonstrate the efficacy of the supplementation of endo-xylanase activity in the production of an improved silage. The data illustrate a substantial increase of lactic acid concentration in the ensiled grass with increasing amounts of supplemented endo-xylanase activity. A slight increase of acetic acid content was observed. This in addition to the increased lactic acid content account for the lower pH and thus a better preservation of the silage. No significant increase in ethanol production (from undesirable yeast fermentation) was observed. Moreover, the data show a significant decrease of cell wall biopolymer parameters (lower crude fiber content, lower Neutral Detergent Fibre content and lower Acid Detergent Fiber content in comparison to the control), resulting in an increase in fermentable sugars available for lactic acid bacteria and for an animal consuming an endo-xylanase treated silage. The pre-digestion of the cell wall material also results in the enhanced digestibility of the ensiled fodder.

**Claims**

1. A silage composition characterized in that it contains more that 50,000 units endo-xylanase activity per kg (fresh weight) fodder, said endo-xylanase activity being supplied to the fodder in a form substantially free of other enzymatic activity.

2. The silage composition of claim 1, further characterized in that the endo-xylanase is obtained from a filamentous fungus.

3. The silage composition of claim 2, further characterized in that the endo-xylanase is obtained from a filamentous fungus selected from the genera consisting of Aspergillus, Disporotrichum, Penicillium, Neurospora, Fusarium and Trichoderma.

4. The silage composition of claim 3, further characterized in that the endo-xylanase is obtained from a filamentous fungus selected from the species consisting of Aspergillus niger, Aspergillus awamori, Aspergillus aculeatus, Aspergillus tubigensis, Disporotrichum dimorphosporum and Trichoderma reesei.

5. The silage composition of claim 1, further characterized in that the endo-xylanase has a pH optimum in the range of pH 3.5 to pH 6.0.

6. The silage composition of claim 1, further characterized in that it further contains an inoculum of a lactic acid bacteria.

7. The silage composition of claim 6, further characterized in that the lactic acid bacteria is selected from the group consisting of Lactobacillus plantarum, Lactobacillus acidophilus, Lactobacillus brevis, Lactobacillus bulgaricus, Lactobacillus fermentum and Lactobacillus lactis.

8. A method of preserving silage characterized in that the silage is supplemented with more than 50,000 units endo-xylanase activity per kg (fresh weight) fodder to increase the amount of water soluble carbohydrates present in the silage for use in the production of lactic acid and acetic acid by lactic acid bacteria, the method being further characterized in that the endo-xylanase is supplied to the fodder in a form substantially free of other enzymatic activity.

9. A method for improving the in vivo digestibility of silage in animals wherein the animal is fed a diet containing silage which has been supplemented with more than 50,000 units endo-xylanase activity per kg (fresh weight) fodder to at least partially degrade the plant cell walls, the method being further characterized in that the endo-xylanase is supplied to the fodder in a form substantially free of other enzymatic activity.

10. A method for the improvement of the nutritional value of silage by supplementing the silage with more than 50,000 units endo-xylanase activity per kg (fresh weight) fodder to release water soluble carbohydrates from the ensiled fodder for utilization by the animal ingesting the silage, the method being further characterized in that the endo-xylanase is supplied to the fodder in a form substantially free of other enzymatic activity.

11. Use of more than 50,000 units endo-xylanase activity per kg (fresh weight) fodder in the preparation of silage, further characterized in that the endo-xylanase is supplied to the fodder in a form substantially free of other enzymatic activity.

**12.** A composition comprising at least 50 wt. % endo-xylanase for use in the treatment of silage, the composition being further characterized in that it is substantially free of other enzymatic activity.

**13.** An animal feedstuff composition comprising a silage treated with more than 50,000 units endo-xylanase per kg (fresh weight) fodder, the composition being further characterized in that the endo-xylanase is supplied to the fodder in a form substantially free of other enzymatic activity.

**Patentansprüche**

**1.** Silofuttermischung, dadurch gekennzeichnet, dass sie mehr als 50 000 Einheiten der Endoxylanase-Aktivität/kg Futter (Frischgewicht) enthält, wobei besagte Endoxylanase-Aktivität dem Futter in von anderer enzymatischer Aktivität nahezu freier Form zugegeben wird.

**2.** Silofuttermischung nach Anspruch 1, dadurch ferner gekennzeichnet, dass die Endoxylanase aus einem Fadenpilz gewonnen wird.

**3.** Silofuttermischung nach Anspruch 2, dadurch ferner gekennzeichnet, dass die Endoxylanase aus einem Fadenpilz gewonnen wird, den man unter den Gattungen Aspergillus, Disporotrichum, Penicillium, Neurospora, Fusarium und Trichoderma auswählt.

**4.** Silofuttermischung nach Anspruch 3, dadurch ferner gekennzeichnet, dass die Endoxylanase aus einem Fadenpilz gewonnen wird, den man unter den Arten Aspergillus niger, Aspergillus awamori, Aspergillus aculeatus, Aspergillus tubigensis, Disporotrichum dimorphosporum und Trichoderma reesei auswählt.

**5.** Silofuttermischung nach Anspruch 1, dadurch ferner gekennzeichnet, dass die Endoxylanase ein pH-Optimum im Bereich von 3,5 bis 6,0 hat.

**6.** Silofuttermischung nach Anspruch 1, dadurch ferner gekennzeichnet, dass sie Impfmaterial einer Milchsäurebakterie enthält.

**7.** Silofuttermischung nach Anspruch 6, dadurch ferner gekennzeichnet, dass man die Milchsäurebakterie unter der Gruppe auswählt, welche aus Lactobacillus plantarum, Lactobacillus acidophilus, Lactobacillus brevis, Lactobacillus bulgaricus, Lactobacillus fermentum und Lactobacillus lactis besteht.

**8.** Verfahren zur Konservierung von Silofutter, dadurch gekennzeichnet, dass dem Silofutter mehr als 50 000 Einheiten der Endoxylanase-Aktivität/kg Futter (Frischgewicht) zugegeben werden, um die im Silofutter vorhandene Menge an wasserlöslichen Kohlehydraten zur Erzeugung von Milchsäure und Essigsäure durch Milchsäurebakterien zu erhöhen, wobei das Verfahren ferner dadurch gekennzeichnet ist, dass die Endoxylanase dem Futter in von anderer enzymatischer Aktivität nahezu freier Form zugegeben wird.

**9.** Verfahren zur Verbesserung der in vivo-Verdaulichkeit von Silofutter bei Tieren, wonach das Tier mit Silofutter enthaltendem Futter gefüttert wird und dem Silofutter mehr als 50 000 Einheiten der Endoxylanase-Aktivität/kg Futter (Frischgewicht) zugegeben werden, um die Zellwand der Pflanzen mindestens teilweise zu zersetzen, wobei das Verfahren ferner dadurch gekennzeichnet ist, dass die Endoxylanase dem Futter in von anderer enzymatischer Aktivität nahezu freier Form zugegeben wird.

**10.** Verfahren zur Verbesserung des Nährwertes von Silofutter, wonach dem Silofutter mehr als 50 000 Einheiten der Endoxylanase-Aktivität/kg Futter (Frischgewicht) zugegeben werden, um die wasserlöslichen Kohlehydrate aus dem silierten Futter freizusetzen, damit sie von dem das Silofutter fressenden Tier verwertet werden, wobei das Verfahren ferner dadurch gekennzeichnet ist, dass die Endoxylanase dem Futter in von anderer enzymatischer Aktivität nahezu freier Form zugegeben wird.

**11.** Verwendung von mehr als 50 000 Einheiten der Endoxylanase-Aktivität/kg Futter (Frischgewicht) bei der Herstellung von Silofutter, dadurch ferner gekennzeichnet, dass die Endoxylanase dem Futter in von anderer enzymatischer Aktivität nahezu freier Form zugegeben wird.

**12.** Mindestens 50 Gew.-% Endoxylanase enthaltende Mischung zur Verwendung als Zugabe zu Silofutter, wobei die Mischung dadurch ferner gekennzeichnet ist, dass sie von anderer enzymatischer Aktivität nahezu frei ist.

**13.** Silofutter enthaltende Tierfuttermischung, wobei das Silofutter mehr als 50 000 Einheiten Endoxylanase-Aktivität/kg Futter (Frischgewicht) enthält und die Mischung dadurch ferner gekennzeichnet ist, dass die Endoxylanase dem Futter in von anderer enzymatischer Aktivität nahezu freier Form zugegeben wird.

**Revendications**

**1.** Composition d'ensilage caractérisée en ce qu'elle contient plus de 50 000 unités d'activité endoxylanase par kg (poids frais) de fourrage, l'activité endoxylanase étant apportée au fourrage sous une forme sensiblement exempte d'autre activité enzymatique.

**2.** Composition d'ensilage suivant la revendication 1, caractérisée, en outre, en ce que l'endoxylanase est obtenue à partir d'un champignon filamenteux.

**3.** Composition d'ensilage suivant la revendication 2, caractérisée, en outre, en ce que l'endoxylanase est obtenue à partir d'un champignon filamenteux choisi parmi les genres consistant en Aspergillus, Disporotrichum, Penicillium, Neurospora, Fusarium et Trichoderma.

**4.** Composition d'ensilage suivant la revendication 3, caractérisée, en outre, en ce que l'endoxylanase est obtenue à partir d'un champignon filamenteux choisi parmi les espèces consistant en Aspergillus niger, Aspergillus awamori, Aspergillus aculeatus, Aspergillus tubigensis, Disporotrichum dimorphosporum et Trichoderma reesei.

**5.** Composition d'ensilage suivant la revendication 1, caractérisée, en outre, en ce que l'endoxylanase a un pH optimal dans l'intervalle de pH 3,5 à pH 6,0.

**6.** Composition d'ensilage suivant la revendication 1, caractérisée, en outre, en ce qu'elle contient un inoculum d'une bactérie à l'acide lactique.

**7.** Composition d'ensilage suivant la revendication 6, caractérisée, en outre, en ce que la bactérie à l'acide lactique est choisie parmi le groupe consistant en Lactobacillus plantarum, Lactobacillus acidophilus, Lactobacillus brevis, Lactobacillus bulgaricus, Lactobacillus fermentum et Lactobacillus lactis.

**8.** Procédé de conservation d'ensilage, caractérisé en ce que l'ensilage est complémenté au moyen de plus de 50 000 unités d'activité endoxylanase par kg (poids frais) de fourrage, pour augmenter la quantité d'hydrates de carbone solubles dans l'eau présents dans l'ensilage, à utiliser dans la production d'acide lactique et d'acide acétique par les bactéries à l'acide lactique, le procédé étant caractérisé, en outre, en ce que l'endoxylanase est introduite dans le fourrage sous une forme sensiblement exempte d'autre activité enzymatique.

**9.** Procédé d'amélioration de la digestibilité in vivo d'un ensilage chez des animaux, où l'animal est nourri d'un régime contenant l'ensilage qui a été complémenté avec plus de 50 000 unités d'activité endoxylanase par kg (poids frais) de fourrage, pour, au moins partiellement, dégrader les parois cellulaires végétales, le procédé étant caractérisé, en outre, en ce que l'endoxylanase est introduite dans le fourrage sous une forme sensiblement exempte d'autre activité enzymatique.

**10.** Procédé d'amélioration de la valeur nutritionnelle d'ensilage en complémentant l'ensilage au moyen de plus de 50 000 unités d'activité endoxylanase par kg (poids frais) de fourrage, pour libérer des hydrates de carbone solubles dans l'eau à partir du fourrage ensilé, à utiliser par l'animal ingérant l'ensilage, le procédé étant caractérisé, en outre, en ce que l'endoxylanase introduite au fourrage est sous une forme sensiblement exempte d'autre activité enzymatique.

**11.** Utilisation de plus de 50 000 unités d'activité endoxylanase par kg (poids frais) de fourrage dans la préparation d'un ensilage, caractérisée, en outre, en ce que l'endoxylanase est introduite au fourrage dans une forme sensiblement exempte d'autre activité enzymatique.

12. Composition comprenant au moins 50% en poids d'endoxylanase, à utiliser dans le traitement d'ensilage, la composition étant caractérisée, en outre, en ce qu'elle est sensiblement exempte d'autre activité enzymatique.

13. Composition alimentaire pour les animaux, comprenant un ensilage traité avec plus de 50 000 unités d'endoxylanase par kg (poids frais) de fourrage, la composition étant caractérisée, en outre, en ce que l'endoxylanase est introduite dans le fourrage sous une forme sensiblement exempte d'autre activité enzymatique.

Figure 1